# EUROPÄISCHE PATENTSCHRIFT

(11) **EP 0 428 970 B1**
(45) Veröffentlichungstag und Bekanntmachung des Hinweises auf die Patenterteilung: **19.01.1994**
(21) Anmeldenummer: 90121604.4
(22) Anmeldetag: 12.11.1990
(51) Int. Cl.: C07C 219/08, C07C 233/38, C07C 231/12, C07C 213/00

(54) **Verfahren zur Herstellung wässriger Lösungen oder Suspensionen von Quaternierungsprodukten von tertiären Aminoalkylestern oder tertiären Aminoalkylamiden der Acryl- oder Methacrylsäure, beispielsweise von Dimethylaminoethylacrylat-Methochlorid**
Process for preparing aqueous solutions or suspensions of quaternization products of tertiary aminoalkylesters or tertiary aminoalkylamides of acrylic or methacrylic acid, for instance of dimethylaminoethyl-acrylate-methochloride
Procédé pour la préparation de solutions ou de suspensions aqueuses de produits de quaternisation d'esters tert-aminoalkyliques, ou d'amides tert-aminoalkyliques de l'acide acrylique ou de l'acide méthycrylique, par example du méthochlorure de l'acrylate de méthylaminoéthylique

(30) Priorität: 21.11.1989 DE 3938528
(43) Veröffentlichungstag der Anmeldung: 29.05.1991
(73) Patentinhaber: BASF Aktiengesellschaft, 67063 Ludwigshafen (DE)
(72) Erfinder: Ascherl, Hermann, Dipl.-Ing., W-6716 Dirmstein (DE); Borho, Klaus, Dr., W-6704 Mutterstadt (DE); Dietrich, Gerhard, Dr., W-6700 Ludwigshafen (DE); Grammatis, Zafirios, Dr., W-6704 Mutterstadt (DE); Niessner, Manfred, Dr., W-6707 Schifferstadt (DE); Sartor, Karl-Heinz, Dipl.-Ing., W-6707 Neuried 2 (DE); Schlindwein, Heinz, W-6701 Birkenheide (DE)

(56) Entgegenhaltungen:
- EP-A- 0 113 038
- EP-A- 0 250 325
- EP-A- 0 329 512
- WO-A-89/07589
- DE-B- 2 848 627

## Beschreibung

Die Erfindung betrifft ein Verfahren zur Herstellung wäßriger Lösungen von Quaternierungsprodukten von tertiären Aminoalkylestern oder tertiären Aminoalkylamiden der Acryl- oder Methacrylsäure durch Reaktion der entsprechenden Ester oder Amide mit einem Alkylierungsmittel, und Anordnungen zur Durchführung des Verfahrens.

Aus der europäischen Patentschrift 0 113 038 ist ein Verfahren zur Herstellung obiger Produkte beschrieben, jedoch unter Verwendung von Lösungsmitteln, beispielsweise Ketonen.

Hierbei ist jedoch folgender Nachteil offensichtlich: Das Lösungsmittel muß von den Produktionsgemischen mit hohem Energieaufwand abgetrennt werden.

Außerdem ist es aus der EP-A 0 250 325, der WO 89/07 589 und der EP-A 329 512 bekannt, diese Reaktion unter jeweils bestimmten Verfahrensbedingungen in Gegenwart von Wasser vorzunehmen. Das Wasser kann jedoch eine teilweise Hydrolyse der Reaktionspartner bewirken, worauf in der DE-B 28 48 627 hingewiesen wird. Nach der Lehre der letztgenannten Patentschrift arbeitet man zwar auch ohne Wasser, dafür aber mit Acrylamid als Zusatzstoff, mit dem das Quaternisierungsprodukt in Mischung anfällt.

Es stellte sich daher die Aufgabe, für die Herstellung obiger Produkte ein Verfahren zu entwickeln, das in Abwesenheit von Lösungsmitteln durchgeführt werden kann.

Diese Aufgabe wird erfindungsgemäß dadurch gelöst, daß die Ausgangsstoffe der Reaktion flüssig zugeführt werden und die Reaktion ohne Verwendung von Lösungsmitteln in einem Rührreaktor mit einem n-fachen stöchiometrischen Überschuß des Alkylierungsmittels, bezogen auf die entsprechenden Ester oder Amide, durchgeführt wird, und wobei die Wasserzugabe zur Herstellung der wäßrigen Lösungen oder Suspensionen nach der Reaktion und vor oder nach der Abtrennung des Überschusses des Alkylierungsmittels erfolgt.

Weitere erfindungsgemäße Merkmale der Erfindung sind Gegenstand der Unteransprüche.

Bei der Quaternierungsreaktion können allgemein tertiäre Aminoalkylester oder tertiäre Aminoalkylamide der Acrylsäure oder Methacrylsäure eingesetzt werden. Besondere Bedeutung für die Herstellung von Homo- und Copolymerisaten haben beispielsweise Di-C₁-C₂-Alkylamino-C₂-C₆-Alkylacrylate oder die entsprechenden Methacrylate sowie Di-C₁-C₂-Alkylamino-C₂-C₆-Alkylacrylamide oder die entsprechenden Methacrylamide. Geeignete Ausgangsprodukte für die Quaternierungsreaktion sind beispielsweise Dimethylaminoethylacrylat, Dimethylaminoethylmethacrylat, Diethylaminoethylacrylat, Diethylaminoethylmethacrylat, Dimethylaminopropylacrylat, Dimethylaminopropylmethacrylat, Diethylaminopropylacrylat, Diethylaminopropylmethacrylat, Dimethylaminoisopropylacrylat, Dimethylaminoisopropylmethacrylat, Dimethylamino-n- oder -iso-butylacrylat, Dimethylamino-n-butylmethacrylat, Dimethylamino-n-pentylacrylat, Dimethylamino-n-pentylmethacrylat, Dimethylaminoneopentylacrylat, Dimethylaminoneopentylmethacrylat, Dimethylaminohexylacrylat und Dimethylaminohexylmethacrylat sowie die entsprechenden basischen Acrylamide und Methacrylamide wie Dimethylaminoethylacrylamid, Dimethylaminoethylmethacrylamid, Diethylaminoethylmethacrylamid, Dimethylaminopropylacrylamid, Dimethylaminopropylmethacrylamid, Dimethylaminoisopropylacrylamid, Dimethylaminoisopropylmethacrylamid, Diethylaminoisopropylacrylamid, Diethylaminoisopropylmethacrylamid, Dimethylamino-n- oder -iso-butylacrylamid, Dimethylamino-n- oder -isobutylmethacrylamid, Diethylamino-n-butylacrylamid, Dimethylamino-neopentylacrylamid, Dimethylamino-neopentylmethacrylamid, Dimethylaminohexylacrylamid und Dimethylaminohexylmethacrylamid.

Als Alkylierungsmittel kommen Alkylhalogenide in Betracht, wobei sich bezüglich der Auswahl der Alkylierungsmittel nur dadurch eine Einschränkung ergibt, daß die verwendeten Alkylierungsmittel unter den gewählten Prozeßbedingungen flüssig sind. Geeignete Alkylierungsmittel sind beispielsweise Alkylhalogenide, wie Methylchlorid, Methylbromid, Methyliodid, Ethylchlorid, Ethylbromid, Ethyliodid, Butylchlorid, Butylbromid, Allylchlorid, Propargylchlorid und Benzylchlorid. Vorzugsweise verwendet man für die Quaternierung der basischen Ester oder Amide Methylchlorid.

Das Verfahren der Erfindung mit den wesentlichen erfindungsgemäßen Merkmalen wird beispielhaft bei der Herstellung einer wäßrigen Lösung von Dimethylaminoethylacrylat-Methochlorid (DMA3-MC) näher beschrieben und zudem in einem Grundfließbild und zwei Verfahrensfließbildern aufgezeigt.

Die Herstellung von DMA3-MC erfolgt nach folgender Reaktionsgleichung:

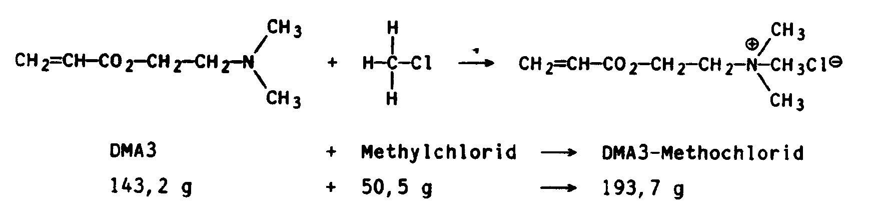

In einer Versuchsanlage, gemäß anschließender Beschreibung, wurde nachstehende Reaktion mehrmalig ausgeführt.

Einsatzstoffe:

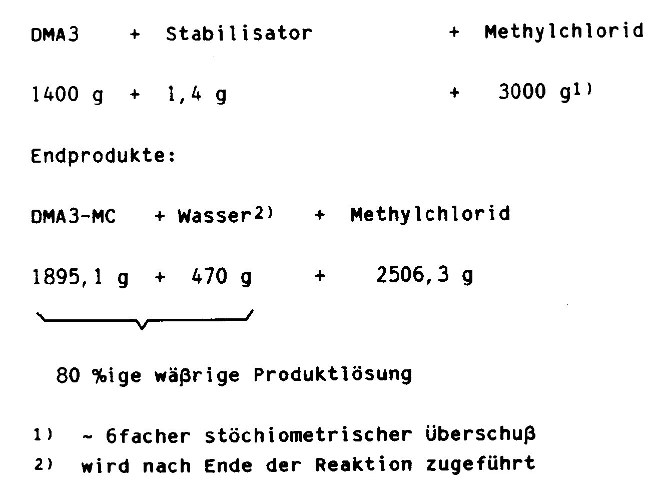

Die einzelnen Verfahrensschritte, Einsatzstoffe, Endprodukte, Drucke und Temperaturen während der einzelnen Verfahrensschritte sind aus dem Grundfließbild zu entnehmen.

Gemäß Verfahrensfließbild 1 bestand die Versuchsanlage aus einem mit einem Heiz-/Kühlmantel ausgerüsteten Rührkessel 1, einem Wärmetauscher 2, einem Behälter 3 und einem Gebläse 4 sowie den Verbindungsrohrleitungen zwischen den einzelnen Apparate-/Maschineneinheiten und notwendigen Meß- und Regeleinrichtungen. Die gesamte Versuchsanlage war bis 10 bar Überdruck ausgelegt und über ein Sicherheitsventil abgesichert.

In den Rührkessel wurden die Einsatzstoffe DMA3 mit dem Stabilisator und Methylchlorid vorgelegt. Der Rührkesselinhalt wurde über den Heizmantel auf 40°C aufgeheizt und der Druck im Rührkessel durch Aufpressen mit Luft auf 10 bar erhöht, wobei im Gasraum des Rührkessels die Luftkonzentration bis auf 50 Vol.% eingestellt wurde. Sauerstoff wirkt als Inhibitor gegen die Polymerisation des DMA3-Methylchlorid-Gemisches. Während der Reaktion wurde durch direkte Siedekühlung mittels des überschüssigen Methylchlorids die Reaktionswärme abgeführt, wobei die Temperatur im Rührkessel auf 40°C gehalten wurde. Die Kondensation des überschüssigen, nicht an der Reaktion teilnehmenden Methylchlorids erfolgte im Wärmetauscher mittels Sole. Hierzu war es notwendig mittels des Gebläses das Luft-/Methylchlorid-Gemisch aus dem Rührkessel abzuziehen, wobei die Kondensation des Methylchlorids im Wärmetauscher erfolgte, während die Luft im Kreis gefahren wurde. Nach Abschluß der Reaktion und ∼ 99,9 %igem DMA3-Umsatz wurde der Rührkesselinhalt durch Zugabe von Wasser auf eine 80 %ige (bezogen auf DMA3-MC/Wasser), homogene, gut rührbare Produktlösung eingestellt. Anschließend wurde das überschüssige Methylchlorid - während Rührens des Rührkesselinhalts - abgedampft, im Wärmetauscher kondensiert und im Behälter aufgefangen. Die nächsten Verfahrensschritte waren Entspannen der Versuchsanlage auf Normaldruck, Neutralisieren im Behälter und Strippen der 80 %igen Produktlösung in bekannter Weise durch Hindurchleiten von Luft durch die Produktlösung im Rührkessel, um die in der Produktlösung verbleibenden Spuren von Methylchlorid zu entfernen.

Gemäß Verfahrensfließbild 2 bestand eine alternative Versuchsanlage aus einem mit einem Heiz-/Kühlmantel ausgerüsteten Schaufeltrockner 1, einem Wärmetauscher 2, einem Behälter 3, einem Gebläse 4, einem mechanischen Förderorgan 5, einem mit Rührer ausgerüsteten Produktbehälter 6 sowie den Verbindungsrohrleitungen zwischen den einzelnen Apparate-/Maschineneinheiten und notwendigen Meß- und Regeleinrichtungen. Die gesamte Versuchsanlage war bis 10 bar Überdruck ausgelegt und über ein Sicherheitsventil abgesichert.

In den Schaufeltrockner wurden die Einsatzstoffe vorgelegt. Der Schaufeltrocknerinhalt wurde über den Heizmantel auf 40°C aufgeheizt und der Druck im Schaufeltrockner durch Aufpressen mit Luft auf 10 bar erhöht, wobei die Luftkonzentration bis auf 50 Vol.% eingestellt wurde. Bei analogem Verfahrensablauf, wie oben beschrieben, wurde nach Abschluß der Reaktion das überschüssige Methylchlorid durch thermische Trocknung - während Drehens des Schaufeltrocknerinhalts - vom Reaktionsprodukt abgetrennt, im Wärmetauscher kondensiert und im Behälter aufgefangen. Nach Entspannen der Versuchsanlage wurde das getrocknete Reaktionsprodukt mittels des mechanischen Förderorgans in den Produktbehälter gefördert und gleichzeitig durch Zugabe von Wasser unter Rühren auf eine 80 %ige, homogene, gut rührbare Produktlösung eingestellt. Der nächste Verfahrensschritt war Strippen der 80 %igen Produktlösung mit Luft in bekannter Weise mittels einer Füllkörperkolonne im Gegenstromverfahren.

Mittels des erfindungsgemäßen Verfahrens - gemäß beiden Versuchsanlagen - wird ein Reaktionsumsatz von bis über 99,9 % - bezogen auf DMA3 - erzielt, und ein Methylchloridrestgehalt im Endprodukt (80 %ige Produktlösung) von bis < 10 ppm erreicht.

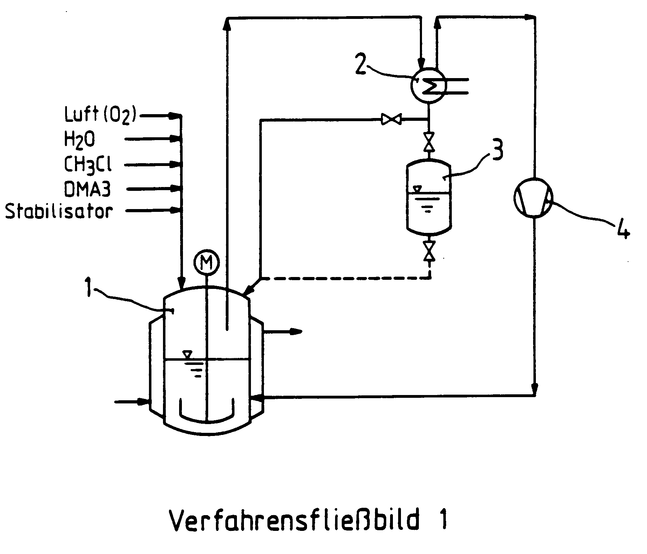

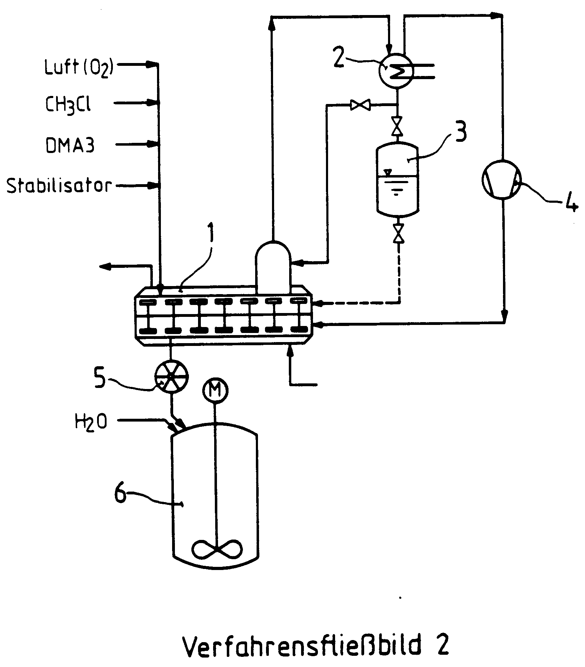

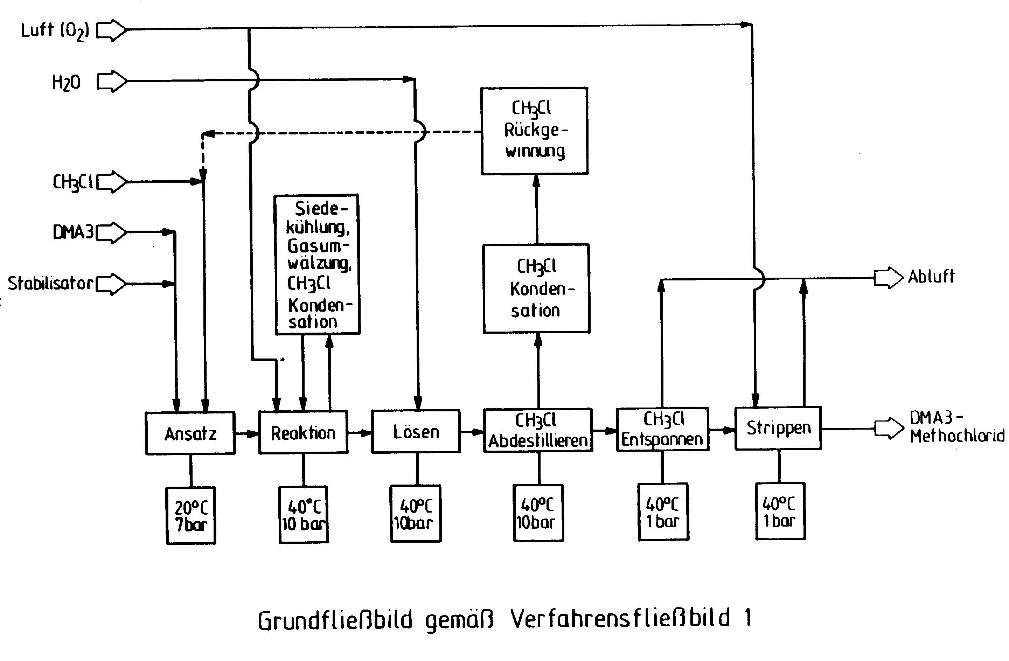

## Patentansprüche

1. Verfahren zur Herstellung wäßriger Lösungen oder Suspensionen von Quaternierungsprodukten von tertiären Aminoalkylestern oder tertiären Aminoalkylamiden der Acryl- oder Methacrylsäure durch Reaktion der entsprechenden Ester oder Amide mit einem Alkylierungsmittel, dadurch gekennzeichnet, daß die Ausgangsstoffe der Reaktion flüssig zugeführt werden und die Reaktion ohne Verwendung von Lösungsmitteln in einem Rührreaktor mit einem n-fachen stöchiometrischen Überschuß des Alkylierungsmittels, bezogen auf die entsprechenden Ester oder Amide, durchgeführt wird, und wobei die Wasserzugabe zur Herstellung der wäßrigen Lösungen oder Suspensionen nach der Reaktion und vor oder nach der Abtrennung des Überschusses des Alkylierungsmittels erfolgt.

2. Verfahren nach Anspruch 1, dadurch gekennzeichnet, daß zur Stabilisierung des Reaktionsgemisches gegen Polymerisation ein wasserlöslicher Stabilisator vor Reaktionsbeginn zugegeben wird.

3. Verfahren nach den Ansprüchen 1 bis 2, dadurch gekennzeichnet, daß die Reaktion mit einem 2- bis 10-fachen stöchiometrischen Überschuß des Alkylierungsmittel durchgeführt wird.

4. Verfahren nach den Ansprüchen 1 bis 3, dadurch gekennzeichnet, daß die Reaktionstemperatur zwischen 0 bis 80°C gehalten wird.

5. Verfahren nach den Ansprüchen 1 bis 4, dadurch gekennzeichnet, daß die Luft- bzw. Sauerstoffkonzentration im Gasraum des Reaktors während der Reaktionsphase bis auf max. 50 bzw. 10 Vol.-% eingestellt wird.

6. Verfahren nach den Ansprüchen 1 bis 5, dadurch gekennzeichnet, daß die Reaktionswärme durch Siedekühlung abgeführt wird, indem das Gasgemisch aus dem Reaktor mittels eines Gebläses umgewälzt wird, der Dampf des Alkylierungsmittels auskondensiert und das Kondensat in den Reaktor zurückgeführt wird.

7. Verfahren nach den Ansprüchen 1 bis 6, dadurch gekennzeichnet, daß das überschüssige Alkylierungsmittel nach Reaktionsende destillativ vom Reaktionsprodukt abgetrennt wird, in einem Wärmetauscher kondensiert und in einem Behälter gesammelt wird, wobei das gesammelte Kondensat dem nächsten Reaktionsansatz wieder zugeführt wird.

8. Verfahren nach den Ansprüchen 1 bis 6, dadurch gekennzeichnet, daß das überschüssige Alkylierungsmittel nach Reaktionsende unter Rühren im Rührreaktor, beispielsweise in einem Schaufeltrockner, durch thermische Trocknung vom Reaktionsprodukt abgetrennt wird, in einem Wärmetauscher kondensiert und in einem Behälter gesammelt wird, wobei das gesammelte Kondensat dem nächsten Reaktionsansatz wieder zugeführt wird.

9. Verfahren nach den vorgehenden Ansprüchen, dadurch gekennzeichnet, daß das getrocknete Reaktionsprodukt mittels eines mechanischen Förderorgans in einen Produktbehälter gefördert wird, und gleichzeitig durch Wasserzugabe unter Rühren im Produktbehälter in eine wäßrige Produktmischung bestimmter Konzentration überführt wird.

10. Verfahren nach den vorhergehenden Ansprüchen, dadurch gekennzeichnet, daß durch Strippung der wäßrigen Produktlösung mit Luft der geforderte Restgehalt an Alkylierungsmittel erzielt wird.

11. Verfahren nach den vorhergehenden Ansprüchen, dadurch gekennzeichnet, daß ein Reaktionsumsatz von bis über 99,9 % und ein Restgehalt an Alkylierungsmittel in der Produktlösung je nach Anforderung bis 10 ppm erzielt wird.

12. Verfahren nach den vorhergehenden Ansprüchen zur Herstellung einer wäßrigen Lösung von Dimethylaminoethylacrylat-Methochlorid (DMA3-MC, C₈H₁₆O₂NCl) aus Dimethylaminoethylacrylat (DMA3, C₇H₁₃O₂N) und Methylchlorid (CH₃Cl), dadurch gekennzeichnet, daß die Ausgangsstoffe (DMA3 und CH₃Cl) der Reaktion flüssig zugeführt werden und die Reaktion in Abwesenheit eines Lösungsmittels mit mindestens einem 4-fachen stöchiometrischen Überschuß des Methylchlorids, bezogen auf Dimethylaminoethylacrylat, durchgeführt wird, und wobei die Wasserzugabe zur Herstellung der wäßrigen Lösung nach der Reaktion und vor der Abtrennung des Überschusses des Methylchlorids erfolgt.

13. Anordnung zur Durchführung des Verfahrens nach den Ansprüchen 1 bis 7 und 10 bis 11, gekennzeichnet durch einen Rührkessel (1) mit Heiz-/Kühlmantel, einen über Kälteträger gekühlten Wärmeaustauscher (2), einen Kondensatsammelbehälter für das Alkylierungsmittel (3), ein Gebläse (4) sowie Verbindungsrohrleitungen zwischen den einzelnen Apparate-/Maschineneinheiten und notwendigen Meß- und Regeleinrichtungen.

14. Anordnung zur Durchführung des Verfahrens nach den Ansprüchen 1 bis 6 und 8 bis 11, gekennzeichnet durch einen Schaufeltrockner (1) mit Heiz-/Kühlmantel, einen über Kälteträger gekühlten Wärmeaustauscher (2), einen Kondensatsammelbehälter für das Alkylierungsmittel (3), ein Gebläse (4), ein mechanisches Förderorgan (5), einen mit Rührer ausgerüsteten Produktbehälter (6) sowie Verbindungsrohrleitungen zwischen den einzelnen Apparate-/Maschineneinheiten und notwendigen Meß- und Regeleinrichtungen.

## Claims

1. A process for the preparation of aqueous solutions or suspensions of quaternization products of tertiary aminoalkyl esters or tertiary aminoalkylamides of acrylic or methacrylic acid by reacting the corresponding esters or amides with an alkylating agent, wherein the starting materials are fed in liquid form into the reaction, and the reaction is carried out without the use of solvents in a stirred reactor with an n-fold stoichiometric excess of the alkylating agent, based on the corresponding esters or amides, the addition of water for the preparation of the aqueous solutions or suspensions being effected after the reaction and before or after the removal of the excess of the alkylating agent.

2. A process as claimed in claim 1, wherein a water-soluble stabilizer is added before the beginning of the reaction in order to stabilize the reaction mixture to polymerization.

3. A process as claimed in claim 1 or 2, wherein the reaction is carried out with a 2- to 10-fold stoichiometric excess of the alkylating agent.

4. A process as claimed in any of claims 1 to 3, wherein the reaction temperature is kept at from 0 to 80 C.

5. A process as claimed in any of claims 1 to 4, wherein the air or oxygen concentration in the gas space of the reactor during the reaction phase is brought to not more than 50 or 10% by volume, respectively.

6. A process as claimed in any of claims 1 to 5, wherein the heat of reaction is removed by evaporative cooling, by circulating the gas mixture from the reactor by means of a fan, condensing out the vapor of the alkylating agent and resetting the condensate into the reactor.

7. A process as claimed in any of claims 1 to 6, wherein the excess alkylating agent is separated from the reaction product by distillation after the end of the reaction, condensed in a heat exchanger and collected in a container, the collected condensate being fed back to the next reaction vessel.

8. A process as claimed in any of claims 1 to 6, wherein the excess alkylating agent is separated from the reaction product by thermal drying after the end of the reaction while stirring in the stirred reactor, for example in a paddle dryer, condensed in a heat exchanger, the collected condensate being fed back to the next reaction batch.

9. A process as claimed in any of the preceding claims, wherein the dried reaction product is transported into a product container by means of a mechanical conveying element and is simultaneously converted into an aqueous product mixture of a certain concentration by adding water while stirring in the product container.

10. A process as claimed in any of the preceding claims, wherein the required residual content of alkylating agent is achieved by stripping the aqueous product solution with air.

11. A process as claimed in any of the preceding claims, wherein a conversion of up to more than 99.9% in the reaction and a residual content of alkylating agent in the product solution of up to 10 ppm, depending on requirements, are achieved.

12. A process as claimed in any of the preceding claims for the preparation of an aqueous solution of dimethylaminoethyl acrylate methochloride (DMA3-MC, C₈H₁₆O₂NCl) from dimethylaminoethyl acrylate (DMA3, C₇H₁₃O₂N) and methyl chloride (CH₃Cl), wherein the starting materials (DMA3 and CH₃Cl) are fed in liquid form to the reaction, and the reaction is carried out in the absence of a solvent with at least a 4-fold stoichiometric excess of the methyl chloride, based on dimethylaminoethyl acrylate, the addition of water for the preparation of the aqueous solution being effected after the reaction and before removal of the excess of methyl chloride.

13. An arrangement for carrying out a process as claimed in any of claims 1 to 7 and 10 to 11, comprising a stirred kettle (1) having a heating/cooling jacket, a heat exchanger (2) cooled by means of a coolant, a condensate collecting container for the alkylating agent (3) and a fan (4) as well as connecting pipelines between the individual apparatus/machine units and necessary measuring and regulating means.

14. An arrangement for carrying out a process as claimed in any of claims 1 to 6 and 8 to 11, comprising a paddle dryer (1) having a heating/cooling jacket, a heat exchanger (2) cooled by means of a coolant, a condensate collecting container for the alkylating agent (3), a fan (4), a mechanical conveyor element (5), a product container (6) equipped with a stirrer, as well as connecting pipelines between the individual apparatus/machine units and necessary measuring and alkylating means.

## Revendications

1. Procédé pour la préparation de solutions ou suspensions aqueuses de produits de quaternisation d'esters aminoalkyliques tertiaires ou d'aminoalkylamides tertiaires de l'acide acrylique ou méthacrylique par réaction des esters ou amides correspondants avec un agent alkylant, caractérisé en ce que l'on envoie les produits de départ à la réaction à l'état liquide et on effectue la réaction sans utiliser de solvant dans un réacteur équipé d'un dispositif d'agitation avec un excès de n fois la quantité stoechiométrique de l'agent alkylant par rapport à l'ester ou amide correspondant, et on ajoute l'eau destinée à la préparation des solutions ou suspensions aqueuse après la réaction et avant ou après la séparation de l'excès d'agent alkylant.

2. Procédé selon la revendication 1, caractérisé en ce que, pour stabiliser le mélange de réaction contre une polymérisation, on ajoute un stabilisant soluble dans l'eau avant le début de la réaction.

3. Procédé selon les revendications 1 à 2, caractérisé en ce que l'on effectue la réaction avec un excès de l'agent alkylant représentant de 2 à 10 fois la quantité stoechiométrique.

4. Procédé selon les revendications 1 à 3, caractérisé en ce que la température de réaction est maintenue entre 0 et 80°C.

5. Procédé selon les revendications 1 à 4, caractérisé en ce que l'on règle la concentration en air ou oxygène dans l'espace des gaz du réacteur jusqu'à un maximum respectif de 50 ou 10 % en volume durant la phase de réaction.

6. Procédé selon les revendications 1 à 5, caractérisé en ce que l'on évacue la chaleur de réaction par refroidissement dû à l'ébullition en faisant circuler le mélange gazeux sortant du réacteur à l'aide d'une soufflante, en condensant les vapeurs de l'agent alkylant et en recyclant le condensat dans le réacteur.

7. Procédé selon les revendications 1 à 6, caractérisé en ce que l'excès d'agent alkylant est séparé par distillation du produit de réaction après la réaction, condensé dans un échangeur de chaleur et recueilli dans un récipient, et le condensat recueilli est recyclé dans la réaction suivante.

8. Procédé selon les revendications 1 à 6, caractérisé en ce que l'excès d'agent alkylant est séparé après la réaction, sous agitation dans un réacteur équipé d'un dispositif d'agitation, par exemple dans un séchoir à palettes, par séchage thermique du produit de réaction, condensé dans un échangeur de chaleur et recueilli dans un récipient, le condensat recueilli étant recyclé dans la réaction suivante.

9. Procédé selon les revendications qui précèdent, caractérisé en ce que le produit de réaction séché est transporté à l'aide d'un organe transporteur mécanique dans un récipient de produit et mis simultanément, par addition d'eau sous agitation dans le récipient de produit, à l'état de mélange de produits aqueux à une concentration déterminée.

10. Procédé selon les revendications qui précèdent, caractérisé en ce que l'on parvient à la teneur résiduelle exigée en agent alkylant en soumettant la solution aqueuse de produit à barbotage d'air.

11. Procédé selon les revendications qui précèdent, caractérisé en ce que dans la réaction, on parvient à un taux de conversion pouvant dépasser 99,9 % et à une teneur résiduelle en agent alkylant dans la solution de produit qui peut tomber à 10 ppm selon les exigences.

12. Procédé selon les revendications qui précèdent, pour la préparation d'une solution aqueuse de méthachlorure de l'acrylate de diméthylaminoéthyle (DMA3-MC, C₈H₁₆O₂NCl) à partir de l'acrylate de diméthylaminoéthyle (DMA3, C₇H₁₃O₂N) et du chlorure de méthyle, (CH₃Cl) caractérisé en ce que l'on envoie les produits de départ (DMA3 et CH₃Cl) à la réaction à l'état liquide et on effectue la réaction en l'absence de solvant avec un excès de chlorure de méthyle représentant au moins 4 fois la quantité stoechiométrique par rapport à l'acrylate de diméthylaminoéthyle, et on ajoute l'eau servant à la préparation de la solution aqueuse après la réaction et avant la séparation de l'excès de chlorure de méthyle.

13. Appareillage pour la mise en oeuvre du procédé selon les revendications 1 à 7 et 10 à 11, caractérisé en ce qu'il comprend un récipient équipé d'un dispositif d'agitation (1) avec double enveloppe de chauffage/refroidissement, un échangeur de chaleur (2) refroidi à l'aide d'un agent réfrigérant, un récipient pour récolte du condensat de l'agent alkylant (3), une soufflante (4) et des conduits de liaison entre les divers unités d'appareillage/machines et les dispositifs nécessaires de mesure et de réglage.

14. Appareillage pour la mise en oeuvre du procédé selon les revendications 1 à 6 et 8 à 11, caractérisé en ce qu'il comprend un séchoir à palettes (1) avec double enveloppe de chauffage/refroidissement, un échangeur de chaleur (2) refroidi à l'aide d'un agent réfrigérant, un récipient de récolte du condensat d'agent alkylant (3), une soufflante (4), un organe transporteur mécanique (5), un récipient de produit (6) équipé d'un agitateur et des conduits de liaison entre les divers unités d'appareillage/machine et les dispositifs nécessaires de mesure et de réglage.
